# EUROPEAN PATENT APPLICATION

(11) **EP 2 048 147 A1**
(43) Date of publication of application: **15.04.2009**
(21) Application number: 07790469.6
(22) Date of filing: 06.07.2007
(51) Int. Cl.: C07F 9/6574, B32B 15/08, B32B 15/092, C08K 5/5313, C08L 63/00, C09K 21/12, H05K 1/03

(54) **PHOSPHORUS-CONTAINING BENZOXAZINE COMPOUND, PROCESS FOR PRODUCTION THEREOF, CURABLE RESIN COMPOSITION, CURED ARTICLE, AND LAMINATE PLATE**

(30) Priority: 20.07.2006 JP 2006198175
(71) Applicant: SHOWA HIGHPOLYMER CO., LTD., Tokyo 105-0012 (JP)
(72) Inventor: TAKAHASHI, Kentarou, Isesaki-shi Gunma 372-0833 (JP); LI, Hui, Isesaki-shi Gunma 372-0833 (JP); KAMATA, Hirotoshi, Isesaki-shi Gunma 372-0833 (JP)
(74) Representative: Gille Hrabal Struck Neidlein Prop Roos
(86) International application number: PCT/JP2007/063599
(87) International publication number: WO 2008/010429

(57) **Abstract**

The present invention is directed to a compound serving as both a flame retardant and a curing agent (crosslinking agent) for curable resin, a method for producing the compound, a flame-retardant curable resin composition containing the compound, and a cured product and a laminated sheet having flame retardancy produced through curing the cured resin composition. The compound has a benzoxazine structure and a phosphine oxide structure in a molecule thereof.

## Description

### Technical Field

The present invention relates to a phosphorus-containing benzoxazine compound which is excellent in heat resistance and water resistance and which is a useful curing agent and flame retardant for epoxy resins and phenolic resins; to a method for producing the compound; to a curable resin composition having flame retardancy and containing the compound; and to a thermally cured product and a laminated sheet having flame retardancy. The compound is suitable as an encapsulant for a semiconductor, a laminated sheet, a coating material, and a composite material, etc.

### Background Art

A variety of epoxy resins and phenolic resins are employed as electric and electronic materials. Parts made of these materials are required to have high flame retardancy, which has been imparted thereto by use of a halogen compound. However, use of halogen compounds has become problematic, with a recent trend for reducing impacts on the environment.
As an alternative technique for imparting flame retardancy, there have been employed phosphorus compounds such as phosphate esters (e.g., triphenyl phosphate) and condensed phosphate esters (e.g., 1,3-phenylene bis(di-2,6-xylenylphosphate). However, when such the phosphorus compounds are added as an additive type flame retardant, the obtained cured product exhibits a drop in heat resistance, particularly in Tg.
In order to solve the problem, there have been proposed techniques (e.g., Patent Documents 1 to 3) employing a reactive phosphorus compound which is produced by reacting an epoxy resin having a novolak epoxy resin of 20% by mass or higher with a quinone compound, and a phosphorus compound (e.g., 9,10-dihydro-9-oxa-10-phosphaphenanthrene-10-oxide or diphenylphosphine oxide), whereby physical properties such as flame retardancy and water resistance are improved. These techniques are based on improvement of heat resistance, flame retardancy, etc. of molded articles through employment of an epoxy resin modified by a phosphorus-compound.
Meanwhile, there has also been proposed use of a compound having a benzoxazine structure as a curing agent for a curable resin composition (e.g., Patent Documents 4 to 7). Also, some proposed techniques employ a phosphorus compound having a benzoxazine structure (e.g., Patent Document 8). However, the amine compound forming the benzoxazine structure is a monoamine compound, which does not ensure satisfactory heat resistance of the cured products.

Patent Document 1: JP 4-11662 A
Patent Document 2: JP 11-279258 A
Patent Document 3: JP 2000-309623 A
Patent Document 4: JP 2001-220455 A
Patent Document 5: JP 2001-329049 A
Patent Document 6: JP 2003-147165 A
Patent Document 7: JP 2004-352670 A
Patent Document 8: JP 2004-528285 A

### Disclosure of the Invention

### Problems to be solved by the Invention

Epoxy resins modified by a phosphorus-compound disclosed in Patent Documents 1 to 3 have drawbacks. For example, the phosphorus content thereof is as low as 2 to 4% by mass, and a large amount of such the epoxy resins are required for providing a phosphorus-containing flame-retardant resin composition. Therefore, physical properties of the resultant cured resin product depend on the physical properties of the epoxy resin modified by a phosphorus-compound. Thus, properties such as glass transition temperature and adhesion are unsatisfactory, which is problematic.
The compounds having a benzoxazine structure and disclosed in Patent Documents 4 to 7 have curing or crosslinking ability, but have no flame retardancy. Therefore, a flame retardant is additionally required.
The compound having a phosphorus-containing benzoxazine structure and disclosed in Patent Document 8 has curing or crosslinking ability and flame retardancy. However, the amine compound forming the benzoxazine structure is a monoamine compound, which does not ensure satisfactory heat resistance of the cured products.
In view of the foregoing, objects of the present invention for solving the problems involved in conventional techniques are to provide a novel compound serving as both a curing agent for curable resin and a flame retardant, a method for producing the compound, a curable resin composition containing the compound, and a cured product and a laminated sheet having flame retardancy produced through curing the resin composition.

### Means for solving the Problems

The present inventors have carried out extensive studies in order to attain the aforementioned objects, and have found that the objects can be attained through employment of a reactive phosphorus compound as a curing agent. It has in a molecule thereof a plurality of phosphorus-containing flame retardant structures and benzoxazine structures each serving as a curing agent.
It can be used as a curing agent for an epoxy resin and/or a resin having a phenolic hydroxyl group, whereby excellent flame retardancy, heat resistance, and water resistance can be imparted to the resin(s) by addition of a small amount of the phosphorus compound, and a cured product having excellent heat and water resistance can be yielded. The present invention has been accomplished on the basis of this finding.
Accordingly, the present invention provides the following:
(1) A phosphorus-containing benzoxazine compound represented by general formula (1):

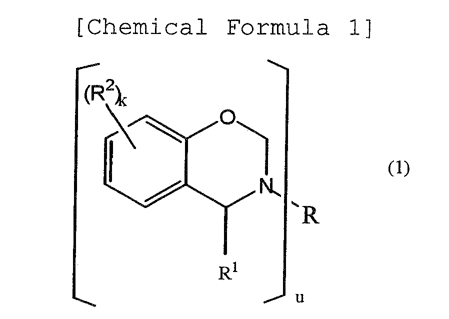

[wherein "R" represents an organic compound residue having a valence of "u"; "u" is an integer of 2 to 10; R¹ represents a group represented by general formula (2):

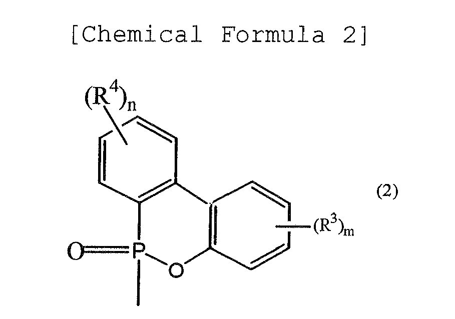

(wherein each of R³ and R⁴ represents a C1 to C6 alkyl group or an optionally substituted aryl group, and each of "m" and "n" is an integer of 0 to 4) or by general formula (3):

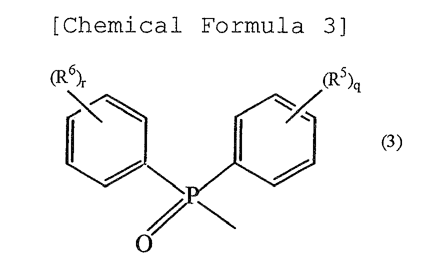

(wherein each of R⁵ and R⁶ represents a C1 to C6 alkyl group or an optionally substituted aryl group, and each of "q" and "r" is an integer of 0 to 5); R² represents a C1 to C6 alkyl group or an optionally substituted aryl group; and "k" is an integer of 0 to 4].
(2) A phosphorus-containing benzoxazine compound as described in the above (1), wherein "u" is 2, and "R" is an optionally substituted alkylene group, an optionally substituted cycloalkylene group, an optionally substituted aralkylene group, an optionally substituted arylene group, or a group represented by general formula (4):

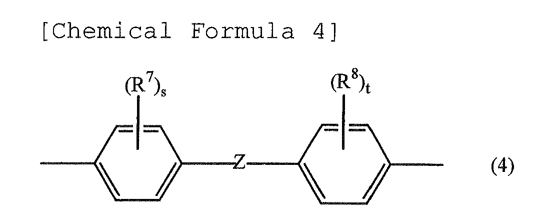

[wherein each of R⁷ and R⁸ represents a C1 to C6 alkyl group or an optionally substituted aryl group; each of "s" and "t" is an integer of 0 to 4; and "Z" represents -CH₂-, -C(CH₃)₂-, an oxygen atom, a sulfur atom, or a sulfone group). (3) A phosphorus-containing benzoxazine compound as described in the above (1) or (2), wherein the compound represented by general formula (1) is any one of the compounds represented by the following general formulas (I) to (III):

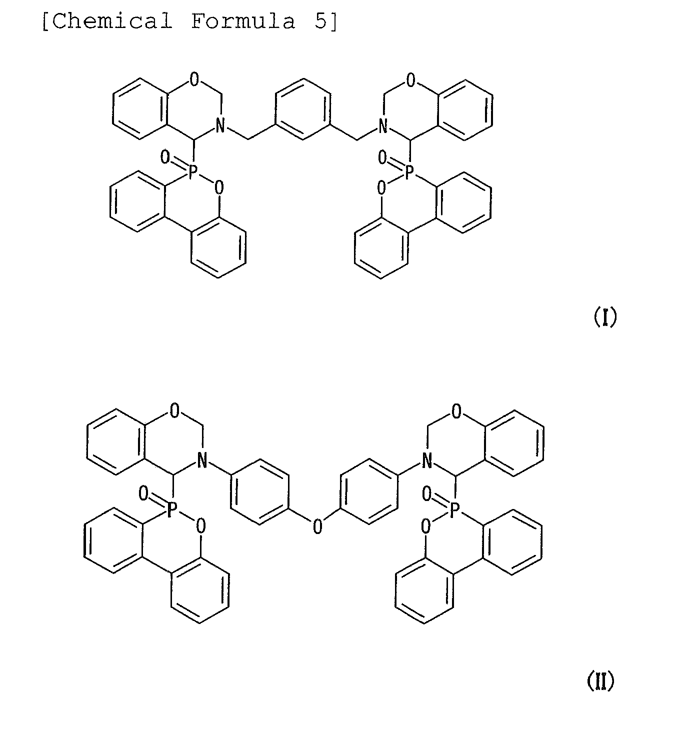

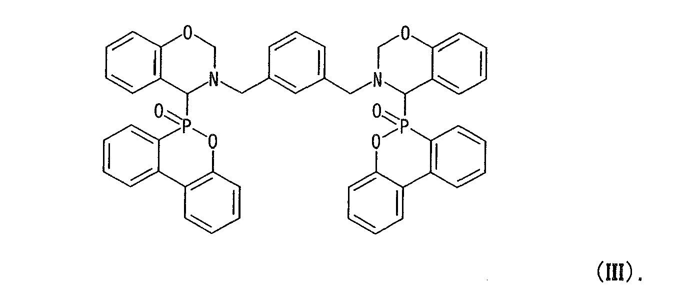

(4) A method for producing a phosphorus-containing benzoxazine compound represented by the general formula (1), characterized by comprising:
reacting a 2-hydroxybenzaldehyde compound represented by general formula (5):

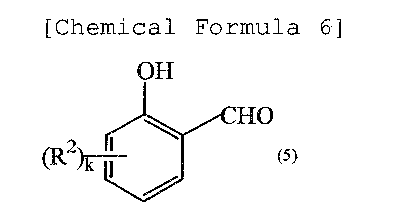

[wherein R² and "k" have the same meanings as defined in the general formula (1)] with an amine compound represented by general formula R(NH₂)ᵤ [wherein "R" and "u" have the same meanings as defined in the general formula (1)], to thereby yield a compound;
reacting, via addition, a phosphorus compound having a structure in which "H" is bound to "P" in a structure represented by general formula (2) or (3) with the yielded compound; and,
subsequently, reacting the addition compound with an aldehyde.
(5) A method for producing a phosphorus-containing benzoxazine compound, characterized by comprising:
   reacting a 2-hydroxybenzaldehyde compound represented by general formula (5):

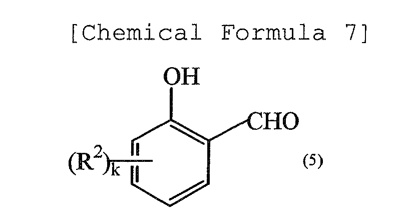

[wherein R² and "k" have the same meanings as defined in the general formula (1)] with a phosphorus compound having a structure in which "H" is bound to "P" in a structure represented by general formula (2) or (3), to thereby yield a compound;
reacting, via addition, an amine compound represented by formula R(NH₂)ᵤ [wherein "R" and "u" have the same meanings as defined in the general formula (1)]; and,
subsequently, reacting the addition compound with an aldehyde.
(6) A curable resin composition having flame retardancy which contains, as essential ingredients, an epoxy resin and/or a resin having a phenolic hydroxyl group, and a phosphorus-containing benzoxazine compound as described in any one of the above (1) to (3).
(7) A cured product formed by thermally curing a curable resin composition having flame retardancy as described in the above (6).
(8) A laminated sheet produced by press-molding a curable resin composition having flame retardancy as described in the above (6) under heating and overlaying thereon with a metal foil.
(9) A laminated sheet as described in the above (8), which is provided with a metal foil on one or both surfaces.

### Effects of the Invention

The curable resin composition of the present invention containing the phosphorus-containing benzoxazine compound and having flame retardancy exhibits high flame retardancy, even though the composition contains no halogen atom, and is excellent in heat resistance and adhesion to copper foil. By virtue of such advantageous properties, the curable resin composition of the present invention having flame retardancy can be suitably employed for producing laminated sheet for electronic boards (printed circuit boards), an encapsulant for semiconductors, etc.

### Best Modes for carrying out the Invention

The present invention will be described hereinafter in detail.
The phosphorus-containing benzoxazine compound of the present invention is represented by the aforementioned formula (1). In formula (1), "u" is an integer of 2 to 10, preferably 2 to 5.
Firstly, a phosphorus-containing benzoxazine compound in which "u" in formula (1) is 2 will be described in detail.
"R" represents an optionally substituted divalent organic compound residue. Examples of the divalent organic compound residue include a C1 to C10 (preferably C1 to C6) alkylene group, an optionally substituted C5 to C15 (preferably C6 to 12) cycloalkylene group, an optionally substituted C7 to C15 (preferably C7 to C12) aralkylene group, and an optionally substituted C6 to C15 (preferably C6 to C12) arylene group. Examples of the substituent include a C1 to C6 (preferably C1 to C2) alkyl group such as methyl or ethyl, a hydroxyl group, an alkoxyl group, and an acyloxyl group.
Examples of the C1 to C10 alkylene group include methylene, ethylene, propanediyls, butanediyls, pentanediyls, hexanediyls, octanediyls, and decanediyls. Examples of the C5 to C15 (preferably C6 to C12) cycloalkylene group include cyclopentylenes, cyclohexylenes, cyclooctylens, and cyclodecylenes. Examples of the C7 to C15 (preferably C7 to C12) aralkylene group include phenylene-methylenes, phenylene-ethylenes, phenylene-propylenes, methylene-phenylenes-methylenes, and naphthylene-methylenes. Examples of the C6 to C15 (preferably C6 to C12) arylene group include phenylenes, naphthylenes, and anthrylenes.
R also includes a structure represented by the aforementioned formula (4).
In formula (4), each of R⁷ and R⁸ represents a C1 to C6 (preferably C1 to C2) alkyl group such as methyl or ethyl, or an optionally substituted aryl group. The aryl group is a C6 to C15 (preferably C6 to C12) aryl group such as phenyl. Each of "s" and "t" is an integer of 0 to 4, preferably 0 to 2. "Z" represents -CH₂-, -C(CH₃)₂-, an oxygen atom, a sulfur atom, or a sulfone group. When "s" is an integer of 2 to 4, a plurality of R⁷s may be identical to or different from each other. When "t" is an integer of 2 to 4, a plurality of R⁸s may be identical to or different from each other.
Specific examples of the divalent group represented by formula (4) include diphenyl ether-4,4'-diyl, diphenyl sulfone-4,4'-diyl, diphenylmethane-4,4'-diyl, and diphenylpropane-4,4'-diyl. Examples of the compound having such a group include 4,4'-diaminodiphenyl ether, 4,4'-diaminodiphenyl sulfone, 4,4'-diaminodiphenylmethane, and 4,4'-diaminodiphenylpropane.

In formula (1), R¹ is represented by the aforementioned formula (2) or (3).
In formula (2), each of R³ and R⁴ represents a C1 to C6 (preferably C1 to C2) alkyl group or an optionally substituted C6 to C15 (preferably C6 to C12) aryl group. Each of "m" and "n" is an integer of 0 to 4, preferably 0 to 2. When "m" and "n" are integers of 2 to 4, a plurality of R³s or R⁴s may be identical to or different from each other.
Examples of the a C1 to C6 alkyl group and the optionally substituted C6 to C15 aryl group include the same alkyl groups and aryl groups as exemplified in relation to "R".
In formula (3), each of R⁵ and R⁶ represents a C1 to C6 (preferably C1 to C2) alkyl group or an optionally substituted C6 to C15 aryl group. Each of "q" and "r" is an integer of 0 to 5, preferably 0 to 2. When "q" and "r" are integers of 2 to 5, a plurality of R⁵s or R⁶s may be identical to or different from each other .
Examples of the a C1 to C6 alkyl group and the C6 to C15 aryl group include the same alkyl groups and aryl groups as exemplified in relation to "R".
In formula (1), R² represents a C1 to C6 (preferably C1 to 2) alkyl group such as methyl or ethyl, or an optionally substituted C6 to C15 (preferably C6 to C12) aryl group. The "k" is an integer of 0 to 4, preferably 0 to 2. When "k" is an integer of 2 to 4, a plurality of R²s may be identical to or different from each other.
Examples of the C1 to C6 alkyl group and the optionally substituted C6 to C15 aryl group include the same alkyl groups and aryl groups as exemplified in relation to R⁷ and R⁸ in formula (4).
When "u" is 2, specific examples of the compound represented by the aforementioned formula (1) include compounds represented by formula (I) to (III).

When "u" is 3 to 10, "R" represents a 3- to 10-valent organic compound residue. Specific examples of such ≥3-valent organic compounds include polymethylene-polyphenylamine. When "u" is 3 to 10, R¹ in the aforementioned formula (1) also is represented by the aforementioned formula (2) or (3). The same substituents as employed in the case where u is 2 are employed in formula (2) or (3).
When "u" is 3 to 10, the same R² as employed in the case where "u" is 2 is employed in the aforementioned formula (1).

Hereinafter, the method for producing the phosphorus-containing benzoxazine compound of the present invention represented by formula (1) will be described.
The phosphorus-containing benzoxazine compound can be readily synthesized from a 2-hydroxybenzaldehyde compound represented by the aforementioned formula (5); an amine compound represented by formula R(NH₂)ᵤ [wherein "R" and "u" have the same meanings as defined in formula (1)]; a phosphorus compound having a structure in which "H" is bound to "P" in the structure represented by formula (2) or (3) (hereinafter, the compound may be referred to simply as "phosphorus compound"); and an aldehyde.
R² and "k" in formula (5) have the same meanings as defined in relation to R² in formula (1).
No particular limitation is imposed on the order of reaction steps. In a production method 1, a 2-hydroxybenzaldehyde compound is reacted with an amine compound, and a phosphorus compound is caused to be reacted (via addition reaction) to the product, followed by reacting with an aldehyde. Alternatively, in a production method 2, a 2-hydroxybenzaldehyde compound is reacted with a phosphorus compound, and an amine compound is caused to be reacted (via addition reaction) to the product, followed by reacting with an aldehyde.
In order to stabilize reaction to suppress side reaction, preferably, reactions in the production method 1 or 2 are generally performed in inert solvent. Such a solvent employed has a boiling point of about 50 to about 250°C. Specific examples include alkanols such as ethanol, propanol (e.g., n-propanol, 2-propanol, and 1-methoxy-2-propanol); aromatic hydrocarbons such as toluene and xylene; alicyclic hydrocarbons such as cyclohexane; cyclic ethers such as tetrahydrofuran and 1,3-dioxorane; ethers such as dimethoxyethylene glycol; esters such as butyl acetate; and amides such as dimethylacetamide.
The amount of solvent with respect to the produced phosphorus-containing benzoxazine compound is about 0.1 to about 5 by mass, preferably about 0.5 to about 2 by mass. When the amount of solvent is adjusted to 0.5 or more, reaction is stabilized to suppress side reaction, whereas when the amount is adjusted to 2 or less, an increase in time and energy required for removing solvent can be prevented.

In the production method 1, a 2-hydroxybenzaldehyde compound and an amine compound are provided such that the mole ratio of aldehyde group to amino group is adjusted to about 1/1, and the mixture is allowed to react in a solvent under reflux with dehydration. Subsequently, a phosphorus compound is added to the reaction product in such an amount that the mole ratio thereof to the formed imino groups is adjusted to about 1/1, and the mixture is allowed to react under reflux. Thereafter, an aldehyde is added to the thus-obtained reaction product in such an amount that the mole ratio of the aldehyde to the secondary amine formed in the reaction is adjusted to about 1/1, and the mixture was is allowed to react under reflux. Finally, solvent is distilled off under reduced pressure and, if required, the product is purified through, for example, washing with water, to thereby remove unreacted substances and by-products.
In the production method 2, a 2-hydroxybenzaldehyde compound and a phosphorus compound are provided such that the mole ratio is adjusted to about 1/1, and the mixture is allowed to react in a solvent under reflux with dehydration. Subsequently, an amine compound is added to the reaction product in such an amount that the mole ratio of amino group to the formed product is adjusted to about 1/1, and the mixture is allowed to react under reflux. Thereafter, an aldehyde is added to the thus-obtained reaction product in such an amount that the mole ratio of the aldehyde to the secondary amine formed in the reaction is adjusted to about 1/1, and the mixture was is allowed to react under reflux.
Finally, solvent is distilled off under reduced pressure and, if required, the product is purified through, for example, washing with water, to thereby remove unreacted substances and by-products.

Examples of the 2-hydroxybenzaldehyde compound represented by formula (5) include 2-hydroxybenzaldehyde, 5-methyl-2-hydroxybenzaldehyde, 2,4-dihydroxybenzaldehyde, 2, 5-dihydroxybenzaldehyde, 2,3-dihydroxybenzaldehyde, and 2,3,4-trihydroxybenzaldehyde. Among them, 2-hydroxy benzaldehyde is preferably employed from the viewpoint of availability.

No particular limitation is imposed on the amine compound, so long as the compound has two or more primary amino groups; i.e., a compound represented by formula R(NH₂)ᵤ
in which "u" is 2 or more. When "u" is 2, specific examples include alkyldiamines such as diaminoethane, diaminopropane, and diaminobutane; aromatic diamines such as p-phenylenediamine, 4,4'-diaminodiphenyl ether, 4,4'-diaminodiphenylmethane, 4,4'-diaminodiphenyl sulfone, dianisidine, and o-tolidine; and m-xylylenediamine. When "u" is 3 or more, examples of the amine compound include polymethylene-polyphnylamine.
When a diamine is employed as the amine compound, there can be produced a compound represented by formula (1) in which "u" is 2, and "R" is a residue formed through removing two amino groups from the used diamine.

No particular limitation is imposed on the phosphorus compound, so long as the compound has a structure in which "H" is bound to "P" in the structure represented by formula (2) or (3). Examples of particularly preferred species thereof include 9,10-dihydro-9-oxa-10-phosphaphenanthrene-10-oxide, diphenylphosphine oxide, bis(2-methylphenyl)phosphine oxide, bis(2,5-dimethylphenyl)phosphine oxide, and bis(2,4,6-trimethylphenyl)phosphine oxide.

Specific examples of the aldehyde include formaldehyde and paraformaldehyde.

The thus-produced phosphorus-containing benzoxazine compound of the present invention represented by the aforementioned formula (1) serves as a flame retardant and a curing agent with respect to a composition containing an epoxy resin and/or a resin having a phenolic hydroxyl group.
In other words, when the benzoxazine ring of the compound is opened through heating, the ring-opened compound is added to the resin having a phenolic hydroxyl group, whereby the resin is cured. At the same time, the formed phenolic hydroxyl group is added to an epoxy group, to thereby cure the epoxy resin.
Thus, when employed in a resin having a phenolic hydroxyl group, an epoxy group, or the like, the phosphorus-containing benzoxazine compound of the present invention is incorporated into the resin skeleton via chemical bonds. Therefore, problems such as drops in heat resistance and glass transition temperature and bleed out of a flame retardant, which would otherwise occur when an additive-type flame retardant is used, can be prevented.

Hereinafter, the flame-retardant curable resin composition, thermally cured product, and laminate sheet of the present invention will be described.
No particular limitation is imposed on the epoxy resin serving as a curable resin, and glycidyl ethers are preferably employed. Examples include bisphenol glycidyl ether, dihydroxybiphenyl glycidyl ether, dihydroxybenzene glycidyl ether, Nitrogen-containing cyclic glycidyl ether, dihydroxynaphthaleneglycidyl ether, phenol-formaldehyde polyglycidyl ether, and polyhydroxyphenol polyglycidyl ether.

Specific examples of bisphenol glycidyl ether include bisphenol A glycidyl ether, bisphenol F glycidyl ether, bisphenol AD glycidyl ether, bisphenol S glycidyl ether, and tetramethylbisphenol A glycidyl ether.

Specific examples of dihydroxybiphenyl glycidyl ether include 4,4'-biphenyl glycidyl ether, 3,3'-dimethyl-4,4'-biphenyl glycidyl ether, and 3,3',5,5'-tetramethyl-4,4'-biphenyl glycidyl ether.

Specific examples of dihydroxybenzene glycidyl ether include resorcin glycidyl ether, hydroquinone glycidyl ether, and isobutylhydroquinone glycidyl ether.

Specific examples of Nitrogen-containing cyclic glycidyl ether include triglycidyl isocyanurate and triglycidyl cyanurate.

Specific examples of dihydroxynaphthalene glycidyl ether include 1,6-dihydroxynaphthalene glycidyl ether and 2,6-dihydroxynaphthalene glycidyl ether.

Specific examples of phenol-formaldehyde polyglycidyl ether include phenol-formaldehyde polyglycidyl ether and cresol-formaldehyde polyglycidyl ether.

Specific examples of polyhydroxyphenol polyglycidyl ether include tris(4-hydroxyphenyl)methane polyglycidyl ether, tris(4-hydroxyphenyl)ethane polyglycidyl ether, tris(4-hydroxyphenyl)propane polyglycidyl ether, tris(4-hydroxyphenyl)butane polyglycidyl ether, tris(3-methyl-4-hydroxyphenyl)methane polyglycidyl ether, tris(3,5-dimethyl-4-hydroxyphenyl)methane polyglycidyl ether, tetrakis(4-hydroxyphenyl)ethane polyglycidyl ether, tetrakis(3,5-dimethyl-4-hydroxyphenyl)ethane polyglycidyl ether, and dicyclopentene-phenol-formaldehyde polyglycidyl ether.
These epoxy resins may be used singly or appropriately in combination of two or more species.

In the present invention, the phosphorus-containing benzoxazine compound and the epoxy resin are used in such a proportion that the amount epoxy group with respect to 1 equivalent of benzoxazine structure is generally adjusted to about 0.5 to about 3 equivalents, preferably about 1.0 to about 2.0 equivalents.
Through adjusting the epoxy group amount to 0.5 equivalent or higher, curing of the epoxy resin can sufficiently proceed, leading to satisfactory mechanical properties, and use of unnecessarily excessive amount of phosphorus-containing benzoxazine compound can be avoided. When the epoxy group amount is adjusted to 3 equivalents or lower, sufficient flame retardancy is ensured.

No particular limitation is imposed on the resin having a phenolic hydroxyl group serving as a curable resin, and bisphenols may be employed. Specific examples include 2,6-dihydroxynaphthalene, 2,2-bis(4-hydroxyphenyl)propane [also called bisphenol A], 2-(3-hydroxyphenyl)-2-(4'-hydroxyphenyl)propane, bis(4-hydroxyphenyl)methane [also called bisphenol F], bis(4-hydroxyphenyl)sulfone [also called bisphenol S], and phenolic resins. Specific examples of phenolic resins include phenol-formaldehyde resin, phenol-aralkyl resin, naphthol-aralkyl resin, and phenol-dicyclopentadiene copolymer resin.
These resins having a phenolic hydroxyl group may be used singly or appropriately in combination of two or more species.

When the phosphorus-containing benzoxazine compound of the present invention is employed as a curing agent for a resin having a phenolic hydroxyl group, the proportion of the amount of phenolic hydroxyl group with respect to 1 equivalent of benzoxazine structure is generally adjusted to about 0.5 to about 5 equivalents, preferably 1.0 to 3.0 equivalents.
Through adjusting the phenolic hydroxy group amount to 0.5 equivalent or higher, curing of the resin having a phenolic hydroxyl group can sufficiently proceed, leading to satisfactory mechanical properties, and use of unnecessarily excessive amount of phosphorus-containing benzoxazine compound can be avoided. When the phenolic hydroxy group amount is adjusted to 5 equivalents or lower, sufficient flame retardancy is ensured.

When the phosphorus-containing benzoxazine compound of the present invention is incorporated into a mixture of an epoxy resin and a resin having a phenolic hydroxyl group, the proportion of the amount epoxy group with respect to the total amount of phenolic hydroxyl group and benzoxazine structure is generally adjusted to about 0.5 to about 3 equivalent, preferably about 1.0 to about 2.0 equivalents. Through adjusting the epoxy group amount to 0.5 equivalent or higher, curing of the epoxy resin and the resin having a phenolic hydroxyl group can sufficiently proceed, leading to satisfactory mechanical properties, and use of unnecessarily excessive amount of the phosphorus-containing benzoxazine compound can be avoided. When the epoxy group amount is adjusted to 3 equivalents or lower, sufficient flame retardancy is ensured.

When the phosphorus-containing benzoxazine compound of the present invention is employed as a curing agent for an epoxy resin and/or a resin having a phenolic hydroxyl group, an additional curing accelerator is preferably employed in combination. The curing accelerator may be selected from those generally employed as curing accelerators for epoxy resin and/or resin having a phenolic hydroxyl group. Examples of such curing accelerators include tertiary amine compounds, quaternary ammonium salts, phosphine compounds, quaternary phosphonium salts, and imidazole compounds.
Examples of tertiary amine compounds which may be used in the invention include 1,8-diazabicyclo[5.4.0]undecene-7, dimethylbenzylamine, and tris(dimethylaminomethyl)phenol.
Examples of such quaternary ammonium salts include tetramethylammonium chloride, tetramethylammonium bromide, benzyltriethylammonium chloride, and benzyltriethylammonium bromide.
Examples of employable phosphine compounds include triphenylphosphine.
Examples of such quaternary phosphonium salts include tetrabutylphosphonium chloride and tetrabutylphosphonium bromide.
Examples of such imidazole compounds include 2-methylimidazole, 2-ethyl-4-methylimidazole, and 1-cyanoethyl-2-undecylimidazole.
These curing accelerators may be used singly or in combination of two or more species.

The curing accelerator is generally added in an amount of about 0.01 to about 10 parts by mass with respect to 100 parts by mass of the resin composition, preferably 0.1 to 5 parts by mass.
In the case where a resin is imparted with flame retardancy by use of the phosphorus-containing benzoxazine compound of the present invention, the benzoxazine is employed in such an amount that phosphorus atoms are generally present in amounts of about 0.1 to about 5.0% by mass in the resin composition, preferably about 0.5 to about 2.0% by mass.
In the case where a resin is imparted with flame retardancy by use of the phosphorus-containing benzoxazine compound of the present invention, if required, an additional flame retardant may be used in combination. Examples of the flame retardant include metal hydroxides such as aluminum hydroxide and phosphorus-containing compounds such as phosphate esters and phosphazene. There may also be employed, as disclosed in the aforementioned JP 11-279258 A, an epoxy resin containing 20% by mass or more a novolak epoxy resin and a compound produced through reaction between a quinone compound and a compound having a structure in which "H" is bound to "P" in the structure represented by formula (2) and/or (3).

The flame-retardant curable resin composition containing the phosphorus-containing benzoxazine compound of the present invention may further contain, in accordance with needs, additives such as a filler, a coupling agent, a lubricant, a mold-releasing agent, a plasticizer, a colorant, and a thickener.

By curing the thus-prepared curable resin composition containing the phosphorus-containing benzoxazine compound of the present invention and having flame retardancy under the following conditions, a thermally cured product is obtained.
The curing temperature and time are generally about 160 to about 240°C and about 30 to about 180 minutes, preferably about 180 to about 220°C and about 60 to about 120 minutes. By controlling the curing temperature to 160°C or higher and the curing time to 30 minutes or longer, curing sufficiently proceeds. By controlling the temperature and time to 240°C or lower and 180 minutes or shorter, discoloring and thermal deterioration (in physical properties) of cured products are prevented, and a drop in productivity is prevented.

When a thermally cured product is produced through thermal reaction of the curable resin composition containing the phosphorus-containing benzoxazine compound of the present invention and having flame retardancy, known molding techniques may be employed. Examples of such molding techniques include melt-cast molding, compression molding (thermally compressing by means of a compression molding machine), transfer molding (injecting a plasticized molding material into a cavity of a metal mold), laminated molding (stacking several prepreg sheets and thermally compressing the laminate for curing to produce a laminated cured product), matched die molding (impregnating a preform with resin, followed by compression molding), SMC method, BMC method, pultrusion molding (unidirectionally impregnating a fiber filament with resin, followed by curing in a die), filament winding (winding resin-impregnated roving by a core), and RIM method.

The curable resin composition containing the phosphorus-containing benzoxazine compound of the present invention and having flame retardancy is more excellent in flame retardancy and heat resistance, as compared with resin compositions employing a conventional flame retardant or based on a conventional flame retardant technique and cured products obtained from such resin compositions. When a metal foil is laminated on the curable resin composition of the present invention, excellent adhesion therebetween can be attained.
By virtue of these excellent characteristics, the phosphorus-containing benzoxazine compound of the present invention and the curable resin composition employing the compound and having flame retardancy can be suitably employed as laminated sheets for electronic boards (printed circuit boards), materials for encapsulating semiconductors, electronic materials for printed circuit boards, etc.
The present invention also provides a laminated sheets, which is formed through compression-molding with heating the aforementioned curable resin composition of the present invention having flame retardancy. The laminated sheets may be provided, on one or both surfaces thereof, with a metal foil. The laminated sheets is suitably employed as substrates for printed circuit boards, etc.
No particular limitation is imposed on the metal forming the metal foil so long as the metal is of general use. Examples of the metal include aluminum, copper, nickel, and alloys thereof. Among them, copper foil and copper-base alloy foil are preferred, from the viewpoints of physical and electric performance, etc.

The curable resin composition containing the phosphorus-containing benzoxazine compound of the present invention may also be employed as a material with which a fiber reinforce substrate (e.g., carbon fiber, glass fiber, aramide fiber, polyester fiber, nylon fiber, or SiC fiber) is impregnated. The amount of fiber reinforce substrate may be appropriately predetermined. For example, the amount is preferably 5 to 500 parts by mass with respect to 100 parts by mass of the resin composition, more preferably 10 to 300 parts by mass. Moreover, the curable resin composition containing the phosphorus-containing benzoxazine compound of the present invention and having flame retardancy can be applied not only to electronic materials but also to automobile parts, OA(office automation)-related parts, etc.

### Examples

The present invention will be described hereinafter in detail by way of Examples, Comparative Examples, and Application Examples, which should not be construed as limiting the invention thereto.

### [Example 1]

An amine compound, 4,4'-diaminodiphenyl ether (200g, 1.0 mol) and 2-hydroxybenzaldehyde (244g, 2.0 mol) were added to 2-propanol (722g), and the mixture was allowed to react for three hours under reflux with dehydration. Subsequently, 9,10-dihydro-9-oxa-10-phosphaphenanthrene-10-oxide (432g, 2.0 mol) was added thereto, and the resultant mixture was allowed to react for three hours under reflux. Subsequently, paraformaldehyde (60g, 2.0 mol) was added thereto, followed by reaction for six hours under reflux. Finally, solvent was distilled off under a reduced pressure, whereby a phosphorus-containing benzoxazine compound (A-1) was yielded as brown solid. Through elemental analysis, mass spectrometry, ¹H-NMR, and infrared absorption spectrometry, the compound A-1 was identified as a compound represented by the aforementioned formula (I).
The phosphorus element content was found to be 7.1% (theoretical value: 7.1%) through elemental analysis, and the molecular weight was found to be 864.1 (calculated: 864.83) through mass spectrometry (M/Z).
¹H-NMR (CDCl₃) absorption peaks were assigned as follows: 4.8 ppm (2H), 5.0 ppm (2H), 5.3 ppm (1H), 5.5 ppm (1H), 6.6 to 7.0 ppm (12H), 7.2 to 7.4 ppm (12H), and 7.7 to 8.0 ppm (4H).
The absorption peaks (cm⁻¹) observed in infrared absorption spectrometry were as follows: 3064, 2924, 2035, 1707, 1607, 1595, 1582, 1561, 1498, 1497, 1449, 1431, 1367, 1309, 1227, 1201, 1147, 1117, 1082, 1036, 1009, 957, 917, 876, 837, 805, 752, 715, and 687.

### [Example 2]

An amine compound, 4,4'-diaminodiphenylmethane (198g, 1.0 mol) and 2-hydroxybenzaldehyde (244g, 2.0 mol) were added to 2-propanol (720g), and the mixture was allowed to react for three hours under reflux with dehydration. Subsequently, 9,10-dihydro-9-oxa-10-phosphaphenanthrene-10-oxide (432g, 2.0 mol) was added thereto, and the resultant mixture was allowed to react for three hours under reflux. Subsequently, paraformaldehyde (60g, 2.0 mol) was added thereto, followed by reaction for six hours under reflux. Finally, solvent was distilled off under reduced pressure, whereby a phosphorus-containing benzoxazine compound (A-2) was yielded as brown solid. Through elemental analysis, mass spectrometry, ¹H-NMR, and infrared absorption spectrometry, the compound A-2 was identified as a compound represented by the aforementioned formula (II).
The phosphorus element content was found to be 7.3% (theoretical value: 7.1%) through elemental analysis, and the molecular weight was found to be 862.1 (calculated: 862.86) through mass spectrometry (M/Z).
¹H-NMR (CDCl₃) absorption peaks were assigned as follows: 3.6 ppm (2H), 4.8 ppm (2H), 5.0 ppm (2H), 5.3 ppm (1H), 5.6 ppm (1H), 6.5 to 7.0 ppm (12H), 7.2 to 7.4 ppm (12H), and 7.7 to 8.0 ppm (4H).
The absorption peaks (cm⁻¹) observed in infrared absorption spectrometry were as follows: 3061, 2928, 1609, 1595, 1584, 1560, 1512, 1490, 1478, 1456, 1448, 1432, 1365, 1293, 1267, 1240, 1205, 1188, 1147, 1119, 1081, 1041, 988, 965, 948, 933, 879, 860, 833, 797, 776, 752, 731, 714, and 688.

### [Example 3]

An amine compound, m-xylylenediamine, (136g, 1.0 mol) and 2-hydroxybenzaldehyde (244g, 2.0 mol) were added to 2-propanol (658 g), and the mixture was allowed to react for three hours under reflux with dehydration. Subsequently, 9,10-dihydro-9-oxa-10-phosphaphenanthrene-10-oxide (432g, 2.0 mol) was added thereto, and the resultant mixture was allowed to react for three hours under reflux. Subsequently, paraformaldehyde (60g, 2.0 mol) was added thereto, followed by reaction for six hours under reflux. Finally, solvent was distilled off under reduced pressure, whereby a phosphorus-containing benzoxazine compound (A-3) was yielded. Through elemental analysis, mass spectrometry, ¹H-NMR, and infrared absorption spectrometry, the compound A-3 was identified as a compound represented by the aforementioned formula (III). The phosphorus content was found to be 7.6% by mass through elemental analysis.

### [Example 4]

An amine compound, polymethylene-polypheylamine (MDA-150, product of Mitsui Chemicals Polyurethanes, amine equivalent: 103), (103g, 1.0 mol as amine equivalent) and 2-hydroxybenzaldehyde (122g, 1.0 mol) were added to 2-propanol (720g), and the mixture was allowed to react for three hours under reflux with dehydration. Subsequently, 9,10-dihydro-9-oxa-10-phosphaphenanthrene-10-oxide (216g, 1.0 mol) was added thereto, and the resultant mixture was allowed to react for three hours under reflux. Subsequently, paraformaldehyde (30g, 1.0 mol) was added thereto, followed by reaction for six hours under reflux. Finally, solvent was distilled off under reduced pressure, whereby a phosphorus-containing benzoxazine compound (A-4) was yielded as brown solid. Through elemental analysis, mass spectrometry, ¹H-NMR, and infrared absorption spectrometry, the compound A-4 was identified as a compound represented by formula (IV) (n=1.6) shown hereinafter. The phosphorus content was found to be 7.0% by mass through elemental analysis.

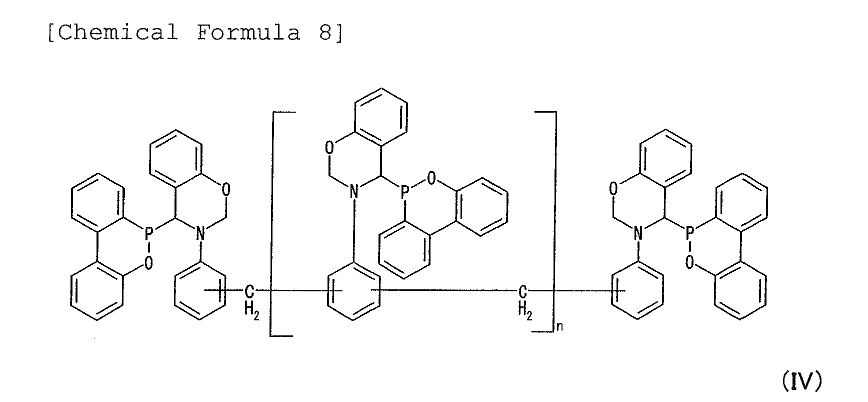

### [Example 5]

9,10-Dihydro-9-oxa-10-phosphaphenanthrene-10-oxide (432g, 2.0 mol) and 2-hydroxybenzaldehyde (244g, 2.0 mol) were added to 1-methoxy-2-propanol (722g), and the mixture was allowed to react at 80°C for three hours with dehydration. Subsequently, an amine compound, 4,4'-diaminodiphenyl ether, (200g, 1.0 mol) was added thereto, and the resultant mixture was allowed to react for three hours under reflux. Subsequently, paraformaldehyde (60g, 2.0 mol) was added thereto, followed by reaction at 80°C for six hours. Finally, solvent was distilled off under reduced pressure, whereby a phosphorus-containing benzoxazine compound (A-5) was yielded as brown solid. Through ¹H-NMR and infrared absorption spectrometry, the compound A-5 was identified as a compound represented by the aforementioned formula (I).
¹H-NMR (CDCl₃) absorption peaks were assigned as follows: 4.8 ppm (2H), 5.0 ppm (2H), 5.3 ppm (1H), 5.5 ppm (1H), 6.6 to 7.0 ppm (12H), 7.2 to 7.4 ppm (12H), and 7.7 to 8.0 ppm (4H).
The absorption peaks (cm⁻¹) observed in infrared absorption spectrometry were as follows: 3064, 2924, 2035, 1708, 1607, 1595, 1582, 1561, 1498, 1497, 1449, 1431, 1367, 1309, 1227, 1201, 1147, 1117, 1082, 1036, 1009, 957, 917, 876, 837, 805, 751, 715, and 687.

### [Example 6]

9,10-Dihydro-9-oxa-10-phosphaphenanthrene-10-oxide (432g, 2.0 mol) and 2-hydroxybenzaldehyde (244g, 2.0 mol) were added to 1-methoxy-2-propanol (658g), and the mixture was allowed to react at 80°C for three hours with dehydration. Subsequently, an amine compound, m-xylylenediamine (136g, 1.0 mol) was added thereto, and the resultant mixture was allowed to react at 80°C for three hours. Subsequently, paraformaldehyde (60g, 2.0 mol) was added thereto, followed by reaction at 80°C for six hours. Finally, 1-methoxy-2-propanol was distilled off under reduced pressure, whereby a phosphorus-containing benzoxazine compound (A-6) was yielded as brown solid. Through ¹H-NMR and infrared absorption spectrometry, the compound A-6 was identified as a compound represented by the aforementioned formula (III).
¹H-NMR (CDCl₃) absorption peaks were assigned as follows: 5.2 ppm (1H), 5.5 ppm (2H), 6.7 ppm (2H), 6.8 ppm (2H), 7.0 to 7.4 ppm (6H), 7.5 ppm (2H), 7.8 ppm (2H), and 8.3 ppm (2H).
The absorption peaks (cm⁻¹) observed in infrared absorption spectrometry were as follows: 3061, 2905, 1594, 1581, 1489, 1472, 1446, 1428, 1365, 1314, 1268, 1257, 1224, 1212, 1202, 1187, 1160, 1148, 1117, 1082, 1042, 1029, 1000, 977, 955, 909, 878, 808, 781, 774, 761, 751, 717, 710, 696, and 687.

### [Comparative Example 1]

An amine compound, aniline, (93g, 1.0 mol) and 2-hydroxybenzaldehyde (122g, 1.0 mol) were added to 2-propanol (459g), and the mixture was allowed to react for three hours under reflux with dehydration. Subsequently, 9,10-dihydro-9-oxa-10-phosphaphenanthrene-10-oxide (216g, 1.0 mol) was added thereto, and the resultant mixture was allowed to react for three hours under reflux. Subsequently, paraformaldehyde (30g, 1.0 mol) was added thereto, followed by reaction for six hours under reflux. Finally, 2-propanol was distilled off under reduced pressure, whereby a phosphorus-containing benzoxazine compound (A-7) was yielded as brown solid. Through elemental analysis, mass spectrometry, ¹H-NMR, and infrared absorption spectrometry, the compound A-7 was identified as a compound represented by formula (V) shown hereinafter. The phosphorus content was found to be 7.1% by mass.

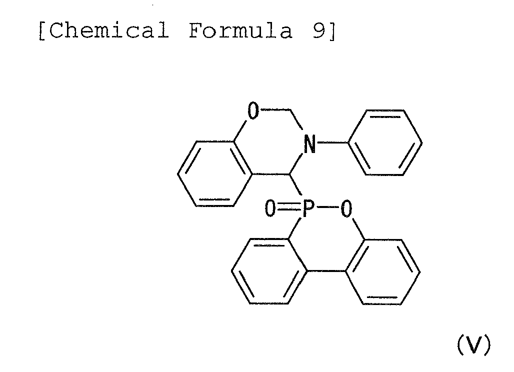

### [Comparative Example 2]

An amine compound, benzylamine (107g, 1.0 mol) and 2-hydroxybenzaldehyde (122g, 1.0 mol) were added to 2-propanol (459g), and the mixture was allowed to react for three hours under reflux with dehydration. Subsequently, 9,10-dihydro-9-oxa-10-phosphaphenanthrene-10-oxide (216g, 1.0 mol) was added thereto, and the resultant mixture was allowed to react for three hours under reflux. Subsequently, paraformaldehyde (30g, 1.0 mol) was added thereto, followed by reaction for six hours under reflux. Finally, 2-propanol was distilled off under reduced pressure, whereby a phosphorus-containing benzoxazine compound (A-8) was yielded as brown solid. Through elemental analysis, mass spectrometry, ¹H-NMR, and infrared absorption spectrometry, the compound A-8 was identified as a compound represented by formula (VI) shown hereinafter. The phosphorus content was found to be 7.0% by mass.

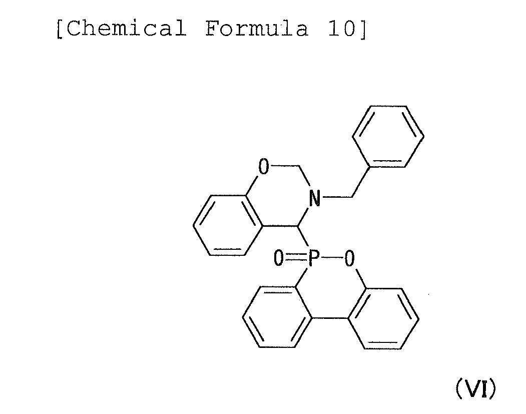

### [Application Examples 1 to 4] and [Comparative Application Examples 1 to 5]

### Formulations of curable resin compositions having flame retardancy

Ingredients shown in Table 1 were dissolved in a solvent at proportions shown in Table 1 through the below-described procedure, to thereby prepare varnishes. Each varnish was cured under the below-described conditions, to thereby prepare double-side-copper-clad laminated sheet pieces. Peel strength, flame retardancy (UL), Tg (DMA method), heat resistance, and solder resistance of the test pieces were measured. Evaluation results are shown in Table 1.
In Table 1, the units "part(s)" and "%" are based on "mass".

[Table 1]

**Table 1-1**

| | Appln. Ex. 1 | Appln. Ex. 2 | Appln. Ex. 3 | Appln. Ex. 4 |
|---|---|---|---|---|
| Epoxy resin (N-680) | 55 | 55 | 57.5 | 55 |
| Epoxy resin (ZX-1548-4) | | | | |
| Phenolic compound (BP-F) | 15 | 15 | 15 | 15 |
| Phenolic compound (LA-7051) | | | | |
| Phosphorus-containing benzoxazine compound (A-1) | 30 | | | |
| The same as above (A-2) | | 30 | | |
| The same as above (A-3) | | | 27.5 | |
| The same as above (A-4) | | | | 30 |
| Comparative Phosphorus-containing benzoxazine compound (A-7) | | | | |
| The same as above (A-8) | | | | |
| Comparative Phosphorus-containing compound (PX-200) | | | | |
| Methoxypropanol | 33 | 33 | 25 | 25 |
| MEK | 33 | 33 | 25 | 25 |
| Curing accelerator (C11Z-CN) | 1 | 1 | 1 | 1 |
| Phosphorus content of curable resin composition (%) | 2.1 | 2.1 | 2.1 | 2.1 |
| Flame retardancy (UL-94) | V-0 | V-0 | V-0 | V-0 |
| Peel strength (kN/m) | 1.6 | 1.4 | 1.5 | 1.5 |
| Tg (DMA, °C) | 170 | 170 | 164 | 176 |
| Heat resistance (°C) | 250 | 250 | 250 | 250 |
| 2% Weight loss temp. (°C) | 368 | 366 | 346 | 370 |
| Solder resistance | Fair | Fair | Fair | Fair |

**Table 1-2**

| | Comp. Appln. Ex.1 | Comp. Appln. Ex. 2 | Comp. Appln. Ex. 3 | Comp. Appln. Ex. 4 | Comp. Appln. Ex. 5 |
|---|---|---|---|---|---|
| Epoxy resin (N-680) | 55 | 55 | | | 53 |
| Epoxy resin (ZX-1548-4) | | | 50 | 50 | |
| Phenolic compound (BP-F) | 15 | 15 | 34 | 20 | 25 |
| Phenolic compound (LA-7051) | | | | 30 | |
| Phosphorus-containing benzoxazine compound (A-1) | | | | | |
| The same as above (A-2) | | | | | |
| The same as above (A-3) | | | | | |
| The same as above (A-4) | | | | | |
| Comparative Phosphorus-containing benzoxazine compound (A-7) | 30 | | | | |
| Comparative Phosphorus-containing benzoxazine compound (A-8) | | 30 | | | |
| Comparative Phosphorus-containing compound (PX-200) | | | | | 22 |
| Methoxypropanol | 25 | 25 | 25 | 25 | 33 |
| MEK | 25 | 25 | 25 | 25 | 33 |
| Curing accelerator (C11Z-CN) | 1 | 1 | 1 | 1 | 1 |
| Phosphorus content of curable resin composition (%) | 2.1 | 2.1 | 2.0 | 2.0 | 2.0 |
| Flame retardancy (UL-94) | V-0 | V-0 | V-0 | V-0 | V-0 |
| Peel strength (kN/m) | 1.6 | 1.5 | 1.4 | 1.3 | 1.5 |
| Tg(DMA,°C) | 149 | 143 | 136 | 152 | 79 |
| Heat resistance (°C) | 240 | 230 | 250 | 240 | 230 |
| 2% weight loss temp. (°C) | 340 | 336 | 376 | 355 | 341 |
| Solder resistance | Fair | Fair | Fair | Fair | Fair |

The following compounds were employed as the epoxy resins and the curing agents in Table 1.

### <Epoxy resins>

(1) Cresol novolak epoxy resin (EPICLON N-680, a product of Dainippon Ink and Chemicals, Inc., epoxy equivalent:
   208g/equivalent)
(2) Novolak epoxy resin modified by a phosphorus compound (ZX-1548-4, a product of Tohto Kasei Co., Ltd., epoxy equivalent=407g/equivalent)

### <Resins having a phenolic hydroxyl group>

(1) Bisphenol F (BP-F, a product of Honshu Chemical Industry Co., Ltd., OH equivalent=100g/equivalent)
(2) Aminotriazine novolak resin (Phenolite LA-7051, a product of Dainippon Ink and Chemicals, Inc., OH equivalent: 124g/equivalent)

### <Phosphorus-containing compounds>

(1) Phosphorus-containing benzoxazine compounds (A-1) to (A-4), (A-7), and (A-8), produced in the Examples and Comparative Examples
(2) 1,3-Phenylenebis(di-2,6-xylenylphosphate) (phosphate ester-based flame retardant, a product of Daihachi Chemical Industry Co, Ltd., PX-200, Phosphorus content: 9.0% by mass)

### <Curing accelerator>

### 1-Cyanoethyl-2-undecylimidazole (a curing accelerator, a product of Shikoku Chemicals Corp., Cresol C11Z-CN)

### [Preparation of varnishes]

Each of the varnishes was prepared by dissolving the ingredients at proportions shown in Table 1 in methoxypropanol-methyl ethyl ketone mixture (solvent); adding a curing accelerator (C11Z-CN) to the solution; and adjusting the non-volatile (N.V.) content of the final curable resin composition to 60% by mass or 66% by mass. The amount of curing accelerator was adjusted to 1 part by mass with respect to 100 parts by mass of resins (an epoxy resin, a resin having a phenolic hydroxyl group, and a curing agent).

### [Conditions under which laminated sheets were produced]

A glass cloth piece (Glass cloth "WE18K105", a product of Nitto Boseki Co., Ltd.) (thickness: about 180 µm) was impregnated with each of the varnishes prepared in Application Examples 1 to 4 and Comparative Application Examples 1 to 5, and solvent was distilled off to dryness. Then, the piece was preliminarily dried at 120°C for 3 min, then at 160°C for 3 min, to thereby prepare a prepreg. A copper foil (thickness: about 18 µm, JTC1/2OZ, a product of Nikko Material) was laminated on each surface of the prepreg, followed by compression-molding at 3.92 MPa and 200°C for 60 min, to thereby prepare a laminated sheet. The thus-produced laminated sheet was found to have a thickness of about 0.2 mm and a resin content of about 40% by mass.

### [Evaluated Physical Properties and Test Conditions]

### (1) Flame retardancy

### Determined in accordance with UL-94 Vertical Burning Test

### (2) Glass transition temperature (Tg)

Determined through the DMA method (rate of temperature elevation: 3°C/min) by means of RTM-1T (a product of ORIENTEC)

### (3) Weight loss initiating temperature

Determined by means of TG/DTA6200 (a product of SII) under a flow of nitrogen at rate of temperature elevation of 10°C/min.

### (4) Elemental analysis

Phosphorus content of a sample was determined by decomposing the sample with sulfuric acid and nitric acid, followed by ICP spectrometry.

### (5) Mass spectrometry

By means of Thermofinnigan LCQ Advantage

### (6) ¹H-Nuclear magnetic resonance spectrometry (¹H-NMR)

By means of JNM-LA300 (a product of JEOL) by use of tetramethylsilane as an internal standard

### (7) Infrared spectrometry

By means of a Fourier transformation infrared spectrometer (Spectrum One, a product of Perkin Elmer)

### (8) Peel strength

Measured in accordance with JIS-C6481

### (9) Heat resistance

Measured in accordance with JIS-C6481, with a testing time was 60 min. The temperature at which all samples (n=3) passed in the test was recorded.

### (10) Solder resistance

Measured in accordance with JIS-C6481. When a sample of a laminated sheet was immersed in a solder at 260°C for 120 seconds, occurrence of swell was visually observed. The sample exhibiting no swell was evaluated as "fair".

As is clear from Table 1, laminated sheets produced through heat curing of the curable resin composition containing the phosphorus-containing benzoxazine compound of the present invention and having flame retardancy exhibit excellent flame retardancy and are excellent in heat resistance and adhesion to a copper foil.

### Industrial Applicability

The curable resin composition containing the phosphorus-containing benzoxazine compound of the present invention and having flame retardancy is suitably used in a field of the electronic material, and particularly suitable for a semiconductor encapsulant, a laminated sheets, a coating material, a composite material, etc.

## Claims

1. A phosphorus-containing benzoxazine compound represented by general formula (1): 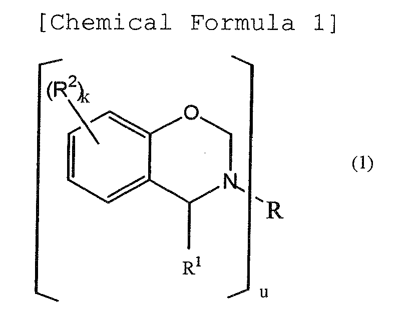 [wherein "R" represents an organic compound residue having a valence of "u"; "u" is an integer of 2 to 10; R¹ represents a group represented by general formula (2): 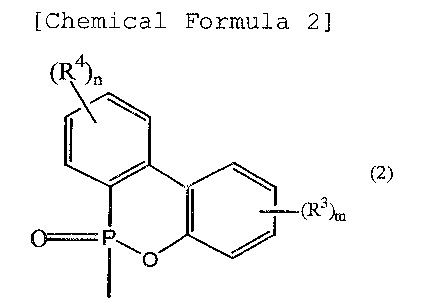 (wherein each of R³ and R⁴ represents a C1 to C6 alkyl group or an optionally substituted aryl group, and each of "m" and "n" is an integer of 0 to 4) or by general formula (3): 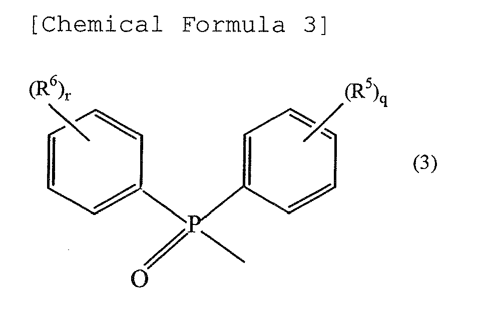 (wherein each of R⁵ and R⁶ represents a C1 to C6 alkyl group or an optionally substituted aryl group, and each of "q" and "r" is an integer of 0 to 5); R² represents a C1 to C6 alkyl group or an optionally substituted aryl group; and "k" is an integer of 0 to 4].

2. A phosphorus-containing benzoxazine compound as claimed in claim 1, wherein "u" is 2, and "R" is an optionally substituted alkylene group, an optionally substituted cycloalkylene group, an optionally substituted aralkylene group, an optionally substituted arylene group, or a group represented by general formula (4): 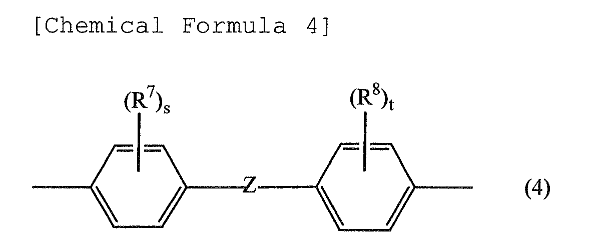 [wherein each of R⁷ and R⁸ represents a C1 to C6 alkyl group or an optionally substituted aryl group; each of "s" and "t" is an integer of 0 to 4; and "Z" represents -CH₂-, -C(CH₃)₂-, an oxygen atom, a sulfur atom, or a sulfone group].

3. A phosphorus-containing benzoxazine compound as claimed in claim 1 or 2, wherein the compound represented by the general formula (1) is any one of the compounds represented by the following general formulas (I) to (III):

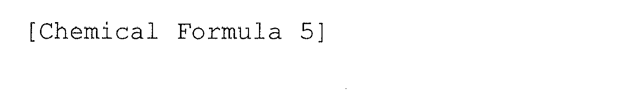

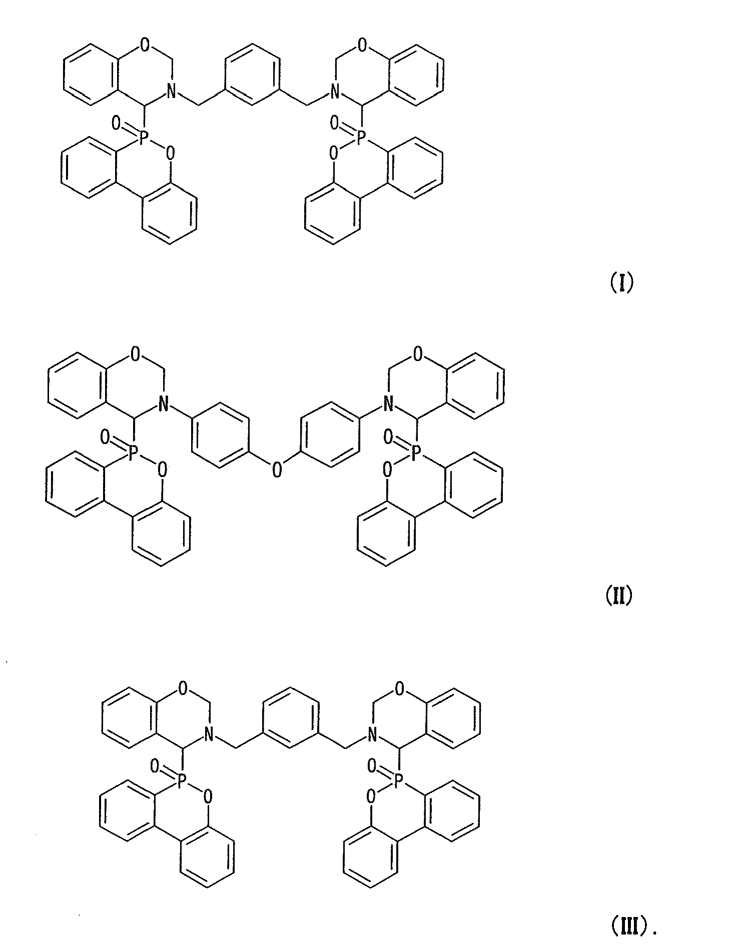

4. A method for producing a phosphorus-containing benzoxazine compound represented by the general formula (1), **characterized by** comprising:
reacting a 2-hydroxybenzaldehyde compound represented by general formula (5): 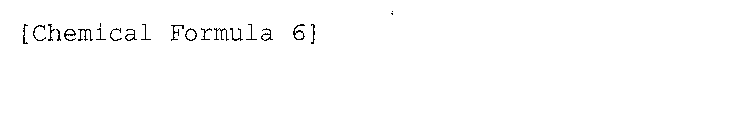 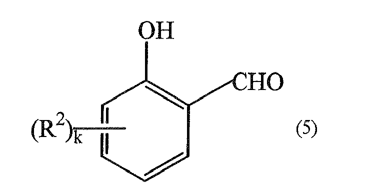 [wherein R² and "k" have the same meanings as defined in the general formula (1)] with an amine compound represented by general formula R(NH₂)ᵤ [wherein "R" and "u" have the same meanings as defined in the general formula (1)], to thereby yield a compound;
reacting, via addition, a phosphorus compound having a structure in which "H" is bound to "P" in a structure represented by general formula (2) or (3) with the yielded compound; and,
subsequently, reacting the addition compound with an aldehyde.

5. A method for producing a phosphorus-containing benzoxazine compound, **characterized by** comprising:
reacting a 2-hydroxybenzaldehyde compound represented by general formula (5): 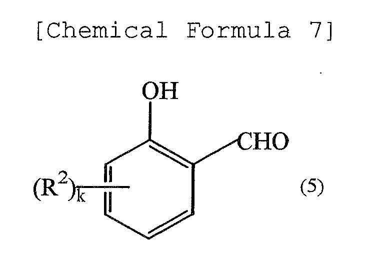 [wherein R² and "k" have the same meanings as defined in the general formula (1)] with a phosphorus compound having a structure in which "H" is bound to "P" in a structure represented by general formula (2) or (3), to thereby yield a compound;
reacting, via addition, an amine compound represented by formula R(NH₂)ᵤ [wherein "R" and "u" have the same meanings as defined in the general formula (1)]; and,
subsequently, reacting the addition compound with an aldehyde.

6. A curable resin composition having flame retardancy which contains, as essential ingredients, an epoxy resin and/or a resin having a phenolic hydroxyl group, and a phosphorus-containing benzoxazine compound as claimed in any one of claims 1 to 3.

7. A cured product formed by thermally curing a curable resin composition having flame retardancy as claimed in claim 6.

8. A laminated sheet produced by compression molding of a curable resin composition having flame retardancy as claimed in claim 6 under heating, and overlaying thereon with a metal foil.

9. A laminated sheet as claimed in claim 8, which is provided with a metal foil on one or both surfaces.
